# EUROPEAN PATENT APPLICATION

(11) **EP 0 839 507 A1**
(43) Date of publication of application: **06.05.1998**
(21) Application number: 96117394.5
(22) Date of filing: 30.10.1996
(51) Int. Cl.: A61F 13/15

(54) **Opening tab for disposable pull-on diapers**

(71) Applicant: THE PROCTER & GAMBLE COMPANY, Cincinnati, Ohio 45202 (US)
(72) Inventor: Schmitz, Christoph Johann, 53881 Euskirchen, Stotzheim (DE); Chan, John Geoffrey, Ashiya-City 659 (JP)
(74) Representative: Canonici, Jean-Jacques

(57) **Abstract**

A disposable pull-on diaper (10) comprising an outer surface, an inner surface, a front portion (11), a rear portion (12), a crotch portion (13), each of said front portion (11) and said rear portion (12) having side panels with side edges (14), and overlapping side seams (15) which join together said side panels of said front portion (11) and said rear portion (12) to form leg openings (16) and a waist area, characterised in that at least one side flap (17) is disposed outboard of each of the side edges (14) of said side panels forming the outermost portion of said overlapping side seams (15).

The side flap is an integral part of the diaper and functions as an opening tab when the diaper is removed by tearing it open.

## Description

### Field of the invention

This invention relates to opening tabs for disposable pull-on diapers.

### Background of the invention

Disposable diapers are well known articles of manufacture which are designed to be worn principally by infants and incontinence sufferers. Such diapers are worn about the lower torso of the wearer and are intended to absorb and contain urine and other bodily discharges, thus preventing the soiling, wetting, or similar contamination of articles (e.g., clothing, bedding, other persons, etc.) that may come into contact with such a diaper in use.

In recent years, disposable diapers in the form of pull-on diapers have emerged on the market and comprise fully elasticised waistbands, elasticised leg cuffs and an absorbent assembly comprising a backsheet, a topsheet and an absorbent core. In particular, disposable pull-on diapers having fixed sides have been popular for use on toilet-training children due to their touch and garment-like appearance. During the toilet training stage, it is important that a child distinguishes a conventional diaper from a pull-on diaper in order to facilitate the transition to washable, reusable cloth underpants. It is also particularly desirable that the fixed sides be separable, so that the pull-on diaper can be easily removed after use. This becomes especially important when the pull-on diaper is soiled and the soilage could be spread and smeared if the diaper has to be removed by sliding it down the length of one or both legs.

It has been documented that the peel forces required to break the overlapping side seams of pull-on diapers can be substantial and it is sometimes difficult to grasp a sufficiently large portion of the side edge of the overlapping side seam panel to tear open the pull-on diaper. There is therefore a risk of the person taking the pull-on diaper off the wearer being hurt, e.g., broken fingernails, or the wearer itself being hurt if too much force is used. Furthermore, the person taking off the pull-on diaper is forced to use both hands to execute the operation. The problem is one which is known in the art but the only solutions proposed hitherto, as far as the present applicants are aware, are those described hereforth.

An example of a disposable undergarment having separable side seams is shown in US 4,205,679. The patent discloses a unitary disposable undergarment for use by toilet training infants or incontinence sufferers having a fabric to adhesive bond which is weaker than the fabric itself thereby permitting the diaper to be torn apart at the seams for easier removal when the undergarment is soiled.

US 4,610,680 shows a disposable training pant that can be opened to facilitate removal from the wearer. In one embodiment, the disposable training pant comprises a tearing strand which has greater cohesive strength than the training pant material, waistband material, or leg band material. Pulling the tearing strand causes the tearing strand to cut through or break the training pant material along its entire length thereby bifurcating it in at least two places. In an alternative embodiment, the training pant can be formed with overlapping flap portions mated by hook and pile fastening strips to permit the disposable training pant to open in at least two places.

US 4,619,649 shows a disposable training pant comprising separable side seams. The seams are formed by overlapping the layers of training pant material which then may either be stitched, heat sealed or adhesively bonded. If the seams are stitched, the training pant is opened by pulling upon the stitching which will separate the seams from the waistband to the leg band. If the seams are heat sealed or adhesively bonded, perforations are used to make this seam separable.

US 4,747,846 teaches a disposable undergarment with manually separable side seams. The side seams are secured by sonic welding, heat sealing or adhesive bonding. The side seam may be an inwardly extending or outwardly extending flange or fin seam, or may be in an overlapping configuration. The side seams are manually separable, or alternatively, the side seams can comprise perforations in the side panels adjacent to the side seam.

Another example of a disposable training pant which can be opened is described in US 4,909,804. The seams of the disposable training pant are sewn using stitching and are inwardly facing. If the garment becomes soiled, the garment is easily torn open at the side seams by breaking the stitching along the entire seam or the sheets of material, because of their fragile nature, can be torn adjacent to the side seam.

The Japanese utility model H6-49292 teaches a disposable diaper with tab portions that are connected to the front part of the diaper to form a pant shape. The tab portions are either made from an easy tear open material or are treated with a releasing agent to permit easy attachment and detachment from the surface of the diaper. In such a manner, the tabs can be used to both open and refasten the diaper.

A further Japanese utility model H6-50623 teaches a disposable pants wherein the front and the back parts of the pants are overlaid and welded together to form outwardly extending fin seams. The welding line is situated inboard of the side edges of the front and back parts such that the protruding parts can form pick-up portions. The welding line is not continuous. In such a manner, finger tips can be inserted in the pant configuration for easy tearage.

US 5,163,932 discloses a disposable wearing article of the pant type wherein the front and rear portions are ultrasonically welded together to form outwardly extending fin seams. The wearing article is characterised by a novel bonding structure to improve ventilation. The bonding line comprises welded and non-welded zones resulting in a pattern that has the effect of a zip when torn open. In order to facilitate the opening of the wearing article, a grip ear is provided, which extends outwards from the side edges of the fin seams. The grip ear can be an integral part of the wearing article or can be manufactured from different material.

US patent application serial number 08/605413 filed 22 February 1996 teaches a disposable pull-on diaper. The application does not disclose an opening tab for easy opening of the overlapping side seams, but rather suggests that the pull-on diaper is removed from the wearer by either pulling it back down over the legs or by tearing the portions of the pull-on diaper adjacent to the overlapping seam panels.

As a result of the above prior art attempts, it has been recognised by those skilled in the art that it would be desirable to provide some device on a pull-on diaper with overlapping side seams that results in a simplification of the opening process. The solution is found to be an opening tab that can easily be cut from the side edges of the side panels forming the outermost portions of the overlapping side seams.

It has now been discovered that the benefits of the present invention range from a simplification of the opening of the side seams on the pull-on diaper; to a very effective and satisfactory gripping portion that permits the peeling of a overlapping side seam even with one hand; to an excellent utilisation of raw materials; and to improved consumer satisfaction and confidence. Furthermore, the inclusion of a process step to allow for the manufacture of the opening tab is straightforward.

### Summary of the invention

A disposable pull-on type diaper is provided with overlapping side seams wherein at least one side flap is disposed outboard of each of the side edges of the side panels forming the outermost portions of the overlapping side seams. The side flap is an integral part of the disposable pull-on diaper and functions as an opening tab.

### Brief description of the drawings

It is believed that the invention will be better understood from the foregoing description in conjunction with the accompanying drawing in which:

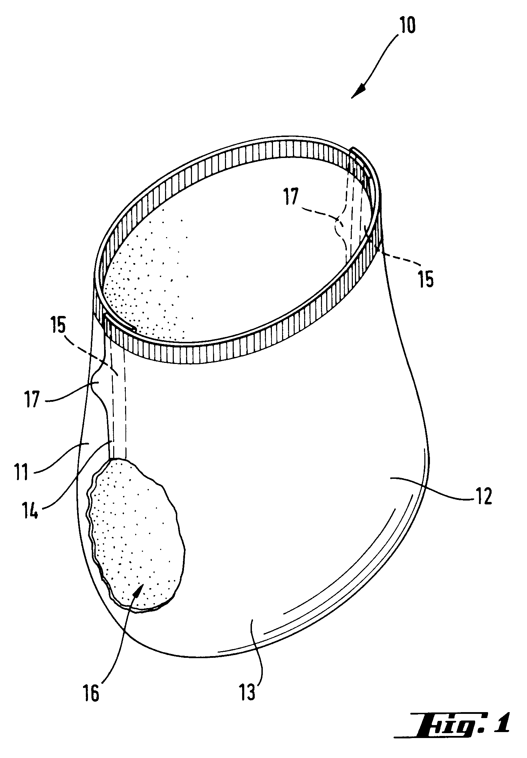

Figure 1 is a perspective view of a disposable pull-on diaper with overlapping side seams and a side flap disposed in an area between the waist and the leg area.

### Detailed description of the invention

As used herein, the term "disposable" describes absorbent articles that are not intended to be laundered or otherwise restored or reused as an absorbent article (i.e., they are intended to be discarded after a single use and, preferably, to be recycled, composted or otherwise disposed of in an environmentally compatible manner. As used herein, the term "diaper" refers to an absorbent article generally worn by infants and sufferers of incontinence that is worn about the lower torso of the wearer. It should be understood, however, that the present invention is also applicable to other pull-on diapers such as training pants, incontinence briefs, feminine hygiene garments or panties, and the like. The term "disposable pull-on diaper" refers to articles of wear, which have a defined waist opening and a pair of leg openings and which are pulled onto the body of the wearer by inserting the legs into the leg openings and pulling the article up over the waist and which are intended to be discarded after a single use. The term "panel" is used herein to denote an area or an element of the pull-on diaper. (While a panel is typically a distinct area or element, a panel may coincide (functionally correspond) somewhat with an adjacent panel.) As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The term "opening tab" refers to a short projecting or protruding device, which can be grasped and can be used to tear open the disposable pull-on diaper. The term "side flap" is synonymous with "opening tab".

Figure 1 is a perspective view of the disposable pull-on diaper 10 of the present invention. The pull-on diaper 10 has an outer surface, an inner surface, a front portion 11, a rear portion 12, a crotch portion 13, each of said front portion 11 and said rear portion 12 having side panels with side edges 14, and overlapping side seams 15 which join together the side panels of the front portion 11 and the rear portion 12 to form leg openings 16 and a waist area. The pull-on diaper 10 thus preferably comprises a chassis layer; an elastically extensible stretch laminate positioned in each side panel of the front portion 11, front stretch laminates; an elastically extensible stretch laminate positioned in each side panel of the rear portion 12, rear stretch laminates; and at least one elasticised waistband positioned in both the front portion 11 and the rear portion 12. The pull-on diaper 10 comprise leg openings 16 which additionally comprise elastic leg features to improve fit at the legs in the crotch portion 13. The pull-on diaper 10 can be furnished with apertures or vents (not shown) in at least the side panels of the pull-on diaper 10 to provide breathability and ventilation.

The pull-on diaper 10 has a crotch portion 13 comprising a main panel and a pair of leg flap panels. The absorbent core is generally positioned within the main panel of the crotch portion 13 since bodily exudates are typically discharged in this area. A leg flap panel extends generally laterally outwardly from and along each side edge of the main panel. Each leg flap panel generally forms at least a portion of the elastic leg feature. The outer surface of the pull-on diaper 10 comprises that portion which is positioned away from the body of the wearer during use. The inner surface of the diaper 10 is opposed to the outer surface and comprises that portion of the diaper which is positioned adjacent to the body of the wearer during use.

Elastically extensible stretch laminates (front stretch laminates and rear stretch laminates) are formed in each side panel of both the front portion 11 and the rear portion 12. Each stretch laminate is mechanically stretched or drawn to allow the stretch laminate to be elastically extensible in at least the lateral direction. (The lateral direction (x direction or width) is defined as the direction parallel to the lateral centreline of the pull-on diaper 10. The side panels are preferably an extension of the chassis layer and other elements such as the topsheet, or any other combination of these elements. In the overlapping side seams 15, the stretch laminate is preferably activated by mechanical stretching to provide additional extensibility in this region. The overlapping side seams 15 may also not be activated by mechanical stretching.

In order to provide the necessary absorbency to contain bodily discharges, the pull-on diaper 10 comprises a liquid pervious topsheet and an absorbent core positioned between the topsheet and the chassis layer. The topsheet is positioned adjacent to the body surface of the absorbent core and is preferably joined to the absorbent core and the chassis layer by attachment means such as those well known in the art. In a preferred embodiment, the topsheet and the chassis layer are indirectly joined together by directly joining them to the absorbent core or the elastic panel members or other elements of the pull-on diaper. The topsheet preferably comprises three distinct layers joined together. A liquid pervious primary layer is positioned over the absorbent core to rapidly absorb liquids into the product. Barrier layers are joined to the primary layer and are preferably drawable, more preferably hydrophobic, to allow the side panels to be mechanically stretched without ripping or tearing while providing barrier cuffs along the sides of the pull-on diaper 10. The elastic leg features preferably comprise a gasketing cuff and a barrier cuff. The gasketing cuff is preferably formed by one or more elastic leg members operatively joined to the chassis layer, the barrier layer, or both, preferably between the chassis layer and the flap portion of the barrier layer in the leg flap panel of the crotch portion 13. The barrier cuff is preferably formed by a flap (the stand-up portion of the barrier layer, closing means for securing the longitudinal ends of the stand-up portion to the primary layer, and an elastic spacing member operatively joined to the stand-up portion.

The primary layer is preferably compliant, soft feeling, and non-irritating to the skin of the wearer. The primary layer is liquid pervious permitting liquids (e.g., urine) to readily penetrate through its thickness. A suitable primary layer may be manufactured from a wide range of materials, such as porous foams; reticulated foams; apertured plastic films or three dimensionally expanded formed films; or woven or nonwoven webs of natural fibers, synthetic fibers, or a combination of natural and synthetic fibers. Preferably, the primary layer is manufactured by Amoco. The primary layer is preferably non-coterminous with the chassis layer so that liquid will not wick along and through the primary layer to the edges of the pull-on diaper 10, so that liquids will not wick underneath and beyond the stand-up barrier cuffs formed by the barrier layers, and so that more drawable materials may be positioned in the side panels to produce stronger stretch laminates. The primary layer preferably overlays a major portion of the body surface of the absorbent core, more preferably all of the body surface area of the absorbent core in at least the crotch portion 13, so that exudates that are discharged into the pull-on diaper 10 penetrate through the primary layer where they are absorbed by the absorbent core. The primary layer extends laterally outwardly toward the side edges of the absorbent core, preferably beyond the side edges of the absorbent core in at least the crotch portion 13. The primary layer, however, terminates inwardly of the leg edges of the crotch portion 13. In the most preferred configurations, the primary layer terminates adjacent the proximal edge of the barrier layer (i.e., the terminating edge of the primary layer is positioned adjacent to the proximal edge) or the terminating edge is positioned remotely from and inboard of the proximal edge. "Adjacent" is used in this context to mean that the primary layer terminates at the proximal edge plus or minus small areas of the primary layer material that may extend inside or beyond the proximal edge due to machine tolerances during manufacture or variations in the area of the primary layer when it is manufactured. In the preferred embodiment of the topsheet, the barrier layers form the elastic leg features (preferably, a gasketing cuff and/or a barrier cuff) and, preferably, a portion of the stretch laminates.

The chassis layer preferably comprises a continuous sheet or web which defines the front portion 11, the rear portion 12, and the crotch portion 13. Thus, the chassis layer is the primary stratum or layer of the pull-on diaper 10. (As used herein, the term "layer" does not necessarily limit the element to a single strata of material in that a layer may actually comprise laminates or combinations of sheets or webs of the requisite type of materials.) The chassis layer has an inner surface and an outer surface. The inner surface and outer surface of the chassis layer correspond in their orientation to the inner surface and the outer surface of the pull-on diaper 10.

In preferred embodiments of the present invention, the chassis layer generally determines the overall shape of the pull-on diaper 10. The chassis layer acts as the main structural layer of the pull-on diaper 10 to which other features may be added or joined. The chassis layer is thus positioned in all or most of the surface area of the pull-on diaper 10, although in certain embodiments certain portions of the chassis layer may be apertured, cut-out or removed ("windowed") to enhance stretchability and/or breathability of the pull-on diaper 10 or features of the pull-on diaper 10 in that area. The chassis layer thus may comprise a continuous sheet or web that does not have "joints" or seams such that forces are distributively transmitted through the entire layer or the chassis layer may comprise a continuous sheet or web that does have "joints" with the elasticised leg cuffs 12. As previously discussed herein, the continuous sheet or web of the chassis layer can comprise a single web of material or a laminate of several continuous webs or layers of different materials. The chassis layer may form the outer surface, the inner surface, or portions of either or both, or may be entirely positioned in the interior of the pull-on diaper 10. The chassis layer preferably forms the outer surface of the pull-on diaper 10 in the crotch portion 13.

Since at least a portion of the chassis layer is subjected to mechanical stretching in order to provide the stretch laminates in the side panels, it is preferably elongatable, more preferably drawable (but not necessarily elastomeric), so that the chassis layer will, upon mechanical stretching, be at least to a degree permanently elongated such that it will not fully return to its original undistorted configuration. The chassis layer may thus comprise any of the materials known for use in absorbent articles such as woven or nonwoven webs; polymeric films such as thermoplastic films of polyethylene, polypropylene, or blends thereof; laminates of such materials; or composite materials. In preferred embodiments, the chassis layer can be subjected to mechanical stretching with minimal or no rupturing or tearing. Therefore, the chassis layer is preferably a polymeric film.

Due to the fact that the chassis layer is preferably a polymeric film, it is also generally impervious to liquids (e.g., urine) so that it may also serve as the component which prevents bodily discharges absorbed and contained in the absorbent core from wetting garments which contact the pull-on diaper 10 such as bed sheets and undergarments (i.e., it acts as the traditional diaper backsheet). If the chassis layer is not liquid impervious, typically an additional layer such as a traditional backsheet should be used behind the absorbent core. The chassis layer may also be breathable (pervious to air or water vapour) if desired. The chassis layer can alternatively comprise breathable materials that are microporous and that are, typically, lower in strength and elongation. An example of such a film is that manufactured by Exxon Chemical Company under the tradename EXXAIRE. Exemplary films for use as the chassis layer of the present invention having relatively good drawability but that are not breathable include polymeric films manufactured by Clopay Corporation of Cincinnati, Ohio under the designation Clopay 1401, or films available from Tredegar of Terre Haute, Indiana, under the designation X-8323 or X-9954.

The size of the chassis layer is dictated by the size of the wearer the pull-on diaper 10 is designed to fit. In a preferred embodiment, the chassis layer has a modified hourglass shape to better fit the wearer. In an embodiment designed to fit large toddlers (about 9 kg to about 15.4 kg), the chassis layer is preferably about 483 millimetres long by about 234 millimetres wide in the front region and the back region and about 165 millimetres wide in the crotch region. The side panels are about 41 millimetres wide, the activated portion of the side panels is about 32 millimetres wide, and the overlapping side seams 15 are about 8.5 millimetres wide. (The actual area of overlap of the overlapping side seams 15 is about 11 millimetres in the embodiment shown herein.) The front portion 11 is about 114 millimetres long, the back portion 12 is about 165 millimetres long, and the crotch portion 13 is about 220 millimetres long.

Seams are formed by bonding together the side panels of the front portion 11 to the side panels of the rear portion 12. The bonding of the seams of the present invention can be by any suitable means well known in the art appropriate for the specific materials employed in the seam panels. Thus, sonic sealing, heat sealing, pressure bonding, adhesive bonding, sewing, autogeneous bonding, and the like may be appropriate techniques. In a preferred embodiment, the side panels of the front portion 11 and the side panels of the rear portion 12 are joined to form overlapping side seams 15. The overlapping side seams 15 are joined by a pattern of heat/pressure or ultrasonic welds. In this particular embodiment, the seams comprises an intermittent pattern of individual bonds grouped in clusters. Potentially strong overlapping side seams may be produced by increasing certain amounts of polymeric material in the side seam panels 15. The amount of polymeric material in the side seams 15 can be increased by using higher basis weight nonwoven materials, thicker plastic films, or by introducing additional layers of materials to the side panels. For example, additional plastic films or nonwoven webs may be joined in the side panels. Alternatively, the layers forming the pull-on diaper 10 may be extended beyond the intended area of seaming and folded back into the side panel to introduce additional strata in the side panels. Examples of these types of seams are discussed in the above-referenced U.S. 5,236,430.

The pull-on diaper 10 comprises a leg area 14 that comprises elasticised leg cuffs 12 for providing improved containment of liquids and other bodily exudates. The elastic leg features provide improved containment of liquids and other body exudates in the crotch portion 13 and about the leg openings 16 in general. Each elastic leg feature may comprise several different embodiments for reducing the leakage of body exudates in the leg flap panels (the elastic leg feature can be and is sometimes also referred to as leg bands, side flaps, barrier cuffs, or elastic cuffs.) US 3,860,003 describes a disposable diaper which provides a contractible leg opening 16 having a side flap and one or more elastic panel members to provide an elasticized leg cuff (gasketing cuff). US 4,909,803 discloses a disposable diaper having "stand-up" elasticized flaps (barrier cuffs) to improve the containment of the leg regions. U.S. 4,695,278 teaches a disposable diaper having dual cuffs including a gasketing cuff and a barrier cuff. US 4,795,454 discloses a disposable diaper having leakage resistant dual cuffs wherein the topsheet stops short of the side edge of the diaper to prevent wicking out to the side of the garment. While each elastic leg feature may be configured so as to be similar to any of the leg bands, side flaps, barrier cuffs or elastic cuffs described above, it is preferred that each elastic leg feature comprise a combination of a gasketing cuff and a barrier cuff. The gasketing cuff and barrier cuffs are preferably formed as shown in US 4,795,454.

The absorbent core is preferably positioned adjacent to the inner surface of the chassis layer and is preferably joined thereto by attachment means such as those well known in the art. For example, the chassis layer may be secured to the absorbent core by a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

The absorbent core may be any absorbent means which is generally compressible, conformable, non-irritating to the skin of the wearer, and capable of absorbing and retaining liquids such as urine and other certain bodily discharges. The absorbent core may be manufactured in a variety of sizes and shapes (e.g., rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core may also be varied (e.g., the absorbent core may have varying caliper zones, hydrophilic gradients, superabsorbent gradients, or lower average density and lower average basis weight acquisition zones; or may comprise one or more layers or structures). The total absorbent capacity of the absorbent core should, however, be compatible with the design loading and the intended use of the pull-on diaper 10. Further, the size and absorbent capacity of the absorbent core may be varied to accommodate wearers ranging from infants through adults.

A preferred embodiment of the absorbent core has an asymmetric, modified hourglass shape and has a body surface toward the body of the wearer (inner surface) and a garment surface opposite the body surface. An exemplary absorbent structure for use as the absorbent core of the present invention that has achieved wide acceptance and commercial success is described in US 5,360,420. Preferably, the absorbent core will comprise an acquisition/distribution layer of chemically stiffened cellulosic fibers and a storage layer positioned beneath the acquisition/distribution layer comprising a mixture of wood pulp fibers and superabsorbent material such as are disclosed in US 4,610,478.

The pull-on diaper 10 can also preferably provided with vents or apertures to permit the passage of air and water vapour to and from the interior of the pull-on diaper 10. In a preferred embodiment, the apertures are positioned in the side panels. In this configuration, bodily discharges are prevented from leaking out of the areas adjacent to the absorbent core but air and water vapour are allowed to be exchanged in the product to ventilate it so that the product does not become excessively wetted by body perspiration and uncomfortable to wear. Vents may additionally be provided in other panels of the pull-on diaper 10 or on certain of the features of the pull-on diaper 10 such as the waistband. The pull-on diaper 10 would preferably have a plurality of vents within the side panels, the vents being arranged in a defined pattern of large and small apertures. Breathability may alternatively be provided by making the materials of the pull-on diaper 10 out of air or vapour permeable materials such as are known in the art. For example, the chassis layer could comprise a breathable (vapour permeable) but liquid impervious plastic film. The elastic panel members may be open material such as foams, scrims, nonwovens, or breathable elastomeric films to further enhance the breathability of the pull-on diaper 10. In a particularly preferred embodiment, the waistband is breathable to allow water vapour to escape from the front portion 11 and the rear portion 12 of the pull-on diaper 10. Breathability may be provided in the waistband by selecting relative breathable materials for its construction and/or by aperturing or venting the waistband such as is discussed herein with respect to the stretch laminates in the side panels. In another embodiment, the waistband may be hydrophobic, hydrophilic, or a combination hydrophobic/hydrophilic member. A hydrophilic waistband may be used to pull moisture away from the skin of the wearer to keep the skin from becoming hydrated. Alternatively, a hydrophobic waistband may be used to prevent fluid absorbed by the diaper 10 from leaking out through the waist opening. A combination hydrophobic/hydrophilic waistband may be used to prevent fluid absorbed by the diaper 10 from leaking out through the waist opening while also pulling moisture away from the skin of the wearer to keep the skin from becoming hydrated.

The present invention also relates to a method for making a disposable pull-on diaper 10. In relation to Figure 1, before joining the side panels of the front portion 11 to the side panels of the rear portion 12, the side flap 17 is cut from the side edges 14 of the side panels forming eventually the outermost portions of the overlapping side seams 15. For this embodiment, the side flap 17 is cut from the side edge 14 of the side panel of the rear portion 12, i.e., the side flap 17 is disposed outboard of the side edges 14 of the side panels. In an alternative embodiment, the side panels of the rear portion 12 may be joined to the side panels of the front portion 11 and for this, the side flap 17 is cut from the side edge 14 of the side panel of the front portion 11. The side flap 17 has preferably a curved shape for easy handling and is preferably disposed along the side edge 14 of the side panel corresponding to either the waist area, the leg area or the area between the waist area and the leg area. No extra material is needed to create the side flap 17. The side flap 17 is thus an integral part of the pull-on diaper 10. An alternative to such an integral structure is a side flap 17 that can be attached to either the side edge 14 of the side panel of the front portion 11 or the side edge 14 of the side panel of the rear portion 12 of the chassis before assemblage using means well known in the art.

The pull-on diaper 10 of the present invention can be applied by a caregiver or be self-applied by the wearer. Typically, the waist opening will be expanded to allow the wearer to insert one of their feet into one of the leg openings 16. The other foot is then inserted into the other leg opening 16. The pull-on diaper 10 is then pulled up over the torso of the wearer into its wearing position. The force wall created by the stretch laminates especially assists in self application of the pull-on diaper 10 by forcing the diaper 10 to be pulled up over the buttocks rather than further expanding. The pull-on diaper 10 is then worn and can contain and hold discharged body exudates. The pull-on diaper 10 is removed from the wearer by tearing open the side flap 17 disposed outboard of the side edges 14 of the side panels forming the outermost portion of the overlapping side seams 15.

### GLOSSARY

- 10: Disposable pull-on diaper
- 11: Front portion
- 12: Rear portion
- 13: Crotch portion
- 14: Side edges
- 15: Overlapping side seams
- 16: Leg openings
- 17: Side flap

## Claims

1. Disposable pull-on diaper (10) comprising an outer surface, an inner surface, a front portion (11), a rear portion (12), a crotch portion (13), each of said front portion (11) and said rear portion (12) having side panels with side edges (14), and overlapping side seams (15) which join together said side panels of said front portion (11) and said rear portion (12) to form leg openings (16) and a waist area,
characterised in that
at least one side flap (17) is disposed outboard of each of the side edges (14) of said side panels forming the outermost portion of said overlapping side seams (15).

2. Disposable pull-on diaper (10) according to claim 1 wherein said side flap (17) is an integral part of said side edges (14).

3. Disposable pull-on diaper (10) according to any of the previous claims wherein said side flap (17) is an opening tab.

4. Disposable pull-on diaper (10) according to any of the previous claims wherein said side flap (17) has a curved shape for easy handling.

5. Disposable pull-on diaper (10) according to any of the previous claims wherein said side flap (17) is disposed along said side edge (14) of said side panel corresponding to either said waist area, said leg area or area between said waist area and said leg area.

6. A method for making a disposable pull-on diaper (10) according to claim 1 wherein said side flap (17) is cut from said side edges (14) of said side panels; and said side panels of said front portion (11) and said rear portion (12) are joined to form overlapping side seams (15).
